# EUROPEAN PATENT APPLICATION

(11) **EP 4 706 519 A1**
(43) Date of publication of application: **11.03.2026**
(21) Application number: 24198475.6
(22) Date of filing: 04.09.2024
(51) Int. Cl.: A61B 5/024, A61B 5/1455, A61B 5/00

(54) **PHOTOPLETHYSMOGRAPHY DEVICE, TISSUE INTERFACE AND SYSTEM FOR DETERMINING VITAL SIGN INFORMATION OF A SUBJECT**

(71) Applicant: Koninklijke Philips N.V., 5656 AG Eindhoven (NL)
(72) Inventor: VUGTS, Marinus Arnoldus Martinus, 5656 AG Eindhoven (NL); SCHWENK, Marcus, 5656 AG Eindhoven (NL)
(74) Representative: Philips Intellectual Property & Standards

(57) **Abstract**

The present invention relates to a photoplethysmography device (10) for acquiring a biosignal of a subject (2), in particular for blood oxygen saturation measurement, the device comprising: a light source (31) configured to emit light signals at one or more wavelengths into tissue of the subject (2); a light detector (32) configured to detect light after travelling from the light source (31) through the tissue of the subject (2) to the light detector (32) along a light path (33) and generate one or more detection signals based on the detected light; wherein the device comprises a tissue interface (40, 50) comprising a first tissue interface region (41, 51) covering the light source (31) and/or the light detector (32) along the light path (33) and adapted to transmit light at the one or more wavelengths, and a second tissue interface region (42, 52) at least partially surrounding the first tissue interface region (41, 51) and adapted to provide relatively less transmission at the one or more wavelengths, wherein the tissue interface (40, 50) comprising the first tissue interface region (41, 51) and the second tissue interface region (42, 52) is a monolithic component are made from the same one single material. The present invention further relates to a corresponding tissue interface (40, 50) and system (1) for determining vital sign information of a subject (2).

## Description

### FIELD OF THE INVENTION

The present invention relates to a photoplethysmography device for acquiring a biosignal of a subject, in particular for blood oxygen saturation measurement. The present invention further relates to a tissue interface and a system for determining vital sign information of a subject.

### BACKGROUND OF THE INVENTION

The concept of providing a photoplethysmography device for acquiring a biosignal for determining vital sign information of a subject, in particular for blood oxygen saturation measurement, is generally known. Blood oxygen saturation measurement is a crucial parameter in assessing how well oxygen is being transported throughout the body. It is typically measured using a non-invasive device called a pulse oximeter, which clips onto a thin part of the body, such as a fingertip, earlobe or nasal alar. The device emits light through the skin and measures the amount of light absorbed by oxygenated and deoxygenated hemoglobin in the blood. Oxygenated hemoglobin absorbs more infrared light and less red light, while deoxygenated hemoglobin absorbs more red light and less infrared light. By analyzing these absorption differences, the pulse oximeter calculates the percentage of hemoglobin molecules bound with oxygen, providing a reading of blood oxygen saturation, or SpO2. Normal SpO2 levels range from 95% to 100%. Levels below 90% may be considered low and may indicate hypoxemia, a condition that may require medical attention. Accurate blood oxygen saturation measurement is essential for monitoring patients with respiratory or cardiac conditions, ensuring they receive appropriate oxygen therapy, and is vital during surgeries and critical care. It provides a quick and reliable way to assess a patient's respiratory function and overall oxygenation status, playing a key role in patient management and treatment.

Disparate bias in blood oxygen saturation measurements refers to the systematic inaccuracies in pulse oximeter readings that disproportionately affect individuals based on their skin pigmentation. Pulse oximeters, which measure blood oxygen levels by analyzing light absorption through the skin, can be less accurate for people with darker skin due to higher melanin levels. Melanin interferes with the light wavelengths used by these devices, often leading to overestimated oxygen saturation readings in individuals with darker skin compared to those with lighter skin. This discrepancy can result in significant clinical implications, such as delayed or insufficient oxygen therapy for patients who need it, thereby exacerbating health disparities. The overestimation of oxygen levels can lead to misinformed clinical decisions, negatively impacting patient care and outcomes. Addressing disparate bias involves improving pulse oximeter technology to better account for variations in skin pigmentation, increasing clinical awareness of this issue, and ensuring that medical devices are tested and validated across diverse populations.

### SUMMARY OF THE INVENTION

It is an object of the present invention to provide a further improved photoplethysmography device. In particular, it would be desirable to provide a photoplethysmography device for acquiring a biosignal of a subject, a tissue interface and a system for determining vital sign information of a subject that can assist in reducing disparate bias in blood oxygen saturation measurements, in particular while at the same time improving user comfort and reducing manufacturing costs.

In a first aspect of the present invention a photoplethysmography (PPG) device for acquiring a biosignal of a subject, in particular for blood oxygen saturation measurement, is presented, the device comprising:
- a light source configured to emit light signals at one or more wavelengths into tissue of the subject;
- a light detector configured to detect light after travelling from the light source through the tissue of the subject to the light detector along a light path and generate one or more detection signals based on the detected light;
- wherein the device comprises a tissue interface comprising a first tissue interface region covering the light source and/or the light detector along the light path and adapted to transmit light at the one or more wavelengths, and a second tissue interface region at least partially surrounding the first tissue interface region and adapted to provide relatively less light transmission (i.e., attenuate light and/or have relatively less transmission compared to the direct or line-of-sight light path from the light source to the light detector through the tissue of the subject) at the one or more wavelengths, wherein the tissue interface comprising the first tissue interface region and the second tissue interface region is a monolithic component made from one single material. In other words, both the first tissue interface region and the second interface region are monolithically made in one continuous piece from the same material. The tissue interface is thus a continuous geometrical component made from one single material. The one single material can be a material having a defined optical non-scattering absorption characteristic for the applicable one or more wavelengths. The one or more detection signals are thus indicative of the biosignal of the subject, for example indicative of a pulse rate or blood oxygen saturation. Examples are provided further below.

In a further aspect of the present invention a tissue interface for a device for acquiring a biosignal of a subject, in particular for blood oxygen saturation measurement, is presented, wherein the tissue interface comprises a first tissue interface region adapted to cover the light source and/or the light detector along the light path and adapted to transmit light at the one or more wavelengths, and a second tissue interface region at least partially surrounding the first tissue interface region and adapted to provide relatively less light transmission (i.e., attenuate light and/or have relatively less transmission compared to the direct or line-of-sight light path from the light source to the light detector through the tissue of the subject) at the one or more wavelengths, wherein the tissue interface comprising the first tissue interface region and the second tissue interface region is a monolithic component made from the one single material. The one single material can be a material having a defined optical non-scattering absorption characteristic for the one or more wavelengths.

In yet a further aspect of the present invention, there is provided a corresponding system for determining vital sign information of a subject, in particular for blood oxygen saturation measurement, the system comprising:
a photoplethysmography device as described herein; and
an analyzer configured to determine vital sign information of the subject from the one or more detection signals.

The present invention is based on the idea of providing a photoplethysmography device, wherein the tissue interface is not simply optimized for maximum transmission at the one or more wavelengths for the PPG measurement, in particular to a tissue interface wherein no separate optical window of a different material with high transmissivity is provided. Instead, the tissue interface is a monolithic component made from one single material, wherein the optical characteristics of the tissue interface are adapted such that, even though the tissue interface is a monolithic component made from one single material, the first tissue interface region is sufficiently transmissive to transmit enough light for the PPG measurement at the one or more wavelengths along the light path from the light source to the light detector. The light path can refer to a direct light path from the light source to the detector through the tissue of the subject when the PPG device is worn by the subject. The first tissue interface region covers the light source and/or the light detector along the light path. However, the second tissue interface region at least partially surrounding the first tissue interface region is attenuating light at the one or more wavelengths outside said (direct) light path to a certain extent. In particular, contributions from stray light which may travel though the upper skin layers with high skin pigmentation can thus be reduced, thereby favorably reducing disparate bias in blood oxygen saturation measurements.

A further advantage over sensors having an interface with two components, wherein one is a window, e.g. a glass window, which lets the light pass and the other component is a surrounding cushion from a different material, is that the manufacturing costs can be reduced. Moreover, an interface with two components may have a step between the window and the surrounding cushion which can reduce user comfort when wearing the PPG device. In contrast thereto, it is suggested to provide a tissue interface that is one single monolithic component consisting of one single material, in particular having a defined optical non-scattering absorption characteristic for the applicable wavelengths in a wavelength range for SpO2 measurements. The proposed tissue interface can be relatively transmissive in the direction of the optical path from the light source through the tissue of the subject to the light detector and can have a relatively lower transmission for an optical path under an angle deviating from the optical path. In particular, the material can be a very high absorbent, semitransparent, non-scattering, optical neutral or controlled density material.

For example, the tissue interface may be adapted such that light emitted from the light source or received by the light detector at an angle of 45° with respect to the direct optical path from the light source through the tissue of the subject to the light detector can be attenuated by the tissue interface by at least 30%, in particular by at least 50%, in particular by at least 70%. The tissue interface can be adapted such that light emitted from the light source or received by the light detector at an angle of 60° with respect to the direct optical path from the light source through the tissue of the subject to the light detector can be attenuated by the tissue interface by at least 30%, in particular by at least 50%, in particular by at least 70%. The first tissue interface region can thus refer to a tissue interface region comprising the direct optical path from the light source through the tissue of the subject to the light detector. The second tissue interface region at least partially surrounds said first tissue interface region and is adapted provide substantial additional attenuation relative thereto.

The tissue interface can be adapted such that an optical path length through the first tissue interface region is shorter than through the second tissue interface region. In other words, an optical path length on the direct optical path from the light source through the tissue of the subject to the light detector through the first tissue interface region is shorter than a second optical path wherein light may propagate through the second tissue interface region. Thereby, light signals at one or more wavelengths through the first tissue interface region experience less absorption and provide a stronger detection signal. In particular, when using tissue interface material with high absorption, unwanted stray light through the second tissue interface region can be attenuated effectively by varying the material thickness and thus the optical path length through the material that attenuates the light. In particular, for a tissue interface with low haze, the optical path length is substantially determined by the material thickness.

Accordingly, a thickness of the second tissue interface region can be larger than a thickness of the first tissue interface region. The thickness can refer to a thickness along an optical path length. This can for example be an optical path based on an angle of incidence under which the light signals are emitted by the light source or received by the light detector.

In an embodiment, the tissue interface can be a monolithic tissue interface cushion. The light source and/or light detector can be embedded in said cushion. The tissue interface can thus be transmitter side or tx tissue interface cushion with the light source embedded in the tissue interface cushion or a receiver side or rx tissue interface cushion with the light detector embedded in the tissue interface cushion. It is also possible to provide a combined cushion wherein both the light source and the detector are embedded in the tissue interface cushion. For example, a tissue interface cushion for a finger clip, nasal or ear lobe PPG device. The tissue interface cushion can be a monolithic tissue interface cushion made from one material, wherein no separate light blocking structures are required in the tissue interface cushion.

The material of the tissue interface can be a material having a transmittance of less than 70 % at 2 mm material thickness at the one or more wavelengths of the light source. In particular, the material of the tissue interface can be a material having a transmittance of less than 50 %, in particular of less than 30 %, at 2 mm material thickness at the one or more wavelengths of the light source. For a PPG measurement using two or more wavelengths, such as a first red wavelength and a second infrared wavelength, the material of the tissue interface can be a material having a transmittance of less than 70 %, in particular of less than 50 %, in particular of less than 30 %, at 2 mm material thickness at only one of the wavelengths, at both of the wavelengths, or if more than two wavelengths are used even at all of the wavelengths used for the PPG measurement. In a refinement, the material of the tissue interface can be a material having a transmittance of less than 70 %, in particular of less than 50 %, in particular of less than 30, at 2 mm material thickness at first wavelength of 662 nm and/or at a second wavelength of 880 nm.

As used herein the transmittance can refer to a photometric luminous transmittance. In the field of photometry, the luminous transmittance of an optical element, here of the material of the tissue interface at 2 mm material thickness, is a measure of the amount of luminous flux or intensity transmitted by said element.

In other words, the material of the tissue interface can be a material having an absorption of at least 30 %, in particular of at least 50 %, in particular of at least 70 %, at 2 mm material thickness at the one or more wavelengths of the light source.

Further, the material of the tissue interface can be a material having a transmittance of at least 5 % at 2 mm material thickness at the one or more wavelengths of the light source. In particular, the material of the tissue interface can be a material having a transmittance of at least 10 % at 2 mm material thickness at the one or more wavelengths of the light source. The transmittance can thus refer to a lower boundary for minimum transmittance such that the material of the tissue interface is sufficiently transparent.

The material of the tissue interface can thus be a very high absorbent, semitransparent material. The inventors recognized that in particular with such a highly absorbent, semitransparent material, it is possible to provide a tissue interface that is sufficiently transparent in a first tissue interface region covering the light source and/or the light detector along the light path and adapted to transmit light at the one or more wavelengths but at the same time sufficiently absorbent to attenuate light or provide relatively less transmission at the one or more wavelengths in a second tissue interface region at least partially surrounding the first tissue interface region such that a PPG measurement can still be performed while at the same time a disparate bias in PPG measurements can be reduced. Thus, there is no need to provide an optical window with a different material. Hence, in essence the surrounding cushion and the material covering the light source and/or the light detector along the light path can be one single component made of the same material having optical properties that are adapted accordingly.

The tissue interface can for example be an injection molded foamskin. A further advantage over a PPG device having an interface with two components can be a better geometry control which can lead to an improved assembly accuracy. This can in turn lead to better sensor result reproducibility.

The light source can be configured to emit light signals at a first red wavelength and at a second infrared wavelength into the tissue of the subject. The material of the tissue interface can be a material, wherein the transmittance at the first red wavelength and at the second infrared wavelength deviates by no more than ±50%, in particular by no more than ±20%, in particular by no more than ±5% from one another. For example, when using a first wavelength of 662 nm and a second wavelength of 880 nm, the transmission for both 662 nm and 880 nm can be within a bandwidth of ±50%, in particular within a bandwidth of ±20%, in particular within a bandwidth of ±5% from one another. The material of the tissue interface can be an optical neutral density or controlled density material. Thereby a homogeneous transmission behavior can be provided. A difference in wavelength might lead to inaccurate measurements. An advantage can thus be an improved measurement accuracy.

The material of the tissue interface can be a material having a haze of less than 50 %, in particular of less than 20 %, in particular of less than 5 %, in particular of less than 2 % at 2 mm material thickness. As used herein haze refers to the amount of light that is subject to wide angle scattering, i.e. scattering at an angle greater than 2.5° from normal in compliance with ASTM D 1003. As used herein, haze can refer to the percentage of incident light scattered by more than 2.5° through the specimen. It measures the milkiness of the material, which is here a sheet having 2 mm thickness. An advantage can be that disparate bias in blood oxygen saturation can be further reduced. The low haze of the material can e.g. ensure that light emitted from the light source or received by the light detector at larger angles with respect to the direct optical path from the light source through the tissue of the subject to the light detector can be sufficiently attenuated by the tissue interface since it propagates through and is attenuated by the tissue interface material rather than being scattered on a shorted optical path onto the light detector.

The tissue interface can be adapted to provide an angle-dependent transmission (and/or an angle-dependent absorption) for light emitted by the light source and/or received by the light detector. In particular, wherein the transmission decreases (or absorption increases) with emission or incidence angle. In particular, wherein the absorption increases progressively with emission or incidence angle. The emission or incidence angle can refer to an angle with respect to an optical axis of light emitted by the light source and/or light received by a detector. For example, emission or incidence angle can refer to an angle with respect to a surface normal of the tissue interface, in particular of the first tissue interface region. The emission or incidence angle can refer to an emission or incidence angle with respect to a direct light path from the light source to the detector. Thereby, light travelling from the light source through the tissue of the subject to the light detector along a direct light path through the first tissue interface region is given more weight than light contributions from the periphery through the second tissue interface region. An advantage can be that disparate bias can be reduced. Moreover, a progressive shape rather than a step-wise increase in material thickness can improve user comfort.

In an embodiment, the light source and/or the light detector can be arranged at a pocket in the tissue interface, wherein said pocket comprises a sloped sidewall. The pocket with the sloped sidewall can be adapted to provide a minimum material thickness at a center of the light source and/or at a center of the light detector. The light source and/or the light detector can thus optionally be arranged in a recess of the tissue interface. Thereby the material thickness in front of the light source and/or the light detector can be reduced in the first tissue interface region, whereas a higher material thickness, thus a respective longer optical path length and thus higher absorption is provided in the second tissue interface region. The pocket can optionally be used to increase an angle-dependent progressiveness of the absorption.

The material of the first tissue interface region and the second tissue interface region can be tinted material, more precisely the same tinted material. For example, the material of the first tissue interface region and the second tissue interface region can be a material having black to grey or grey color, in particular the same tinted material having black to grey or grey color. An advantage can be that the tissue interface can be manufactured efficiently. A further advantage can be favorable biocompatibility. Moreover, the optical properties can be tailored so as to provide a one or more of a transmittance, absorption and/or haze as described herein.

In an embodiment the material is silicone. In an embodiment the material is a thermoplastic elastomer. In an embodiment the material is a thermoset. In an embodiment the material is any compound of silicone, a thermoplastic elastomer and/or a thermoset.

The device can be a transmission type photoplethysmography device. In a transmission type PPG device, the light source and light detector can be arranged on opposite sides of a tissue region of the subject, for example on opposite sides of a finger, ear lobe or nasal alar. In particular, a direct or line-of-sight light path from the light source through the tissue of the subject to the light detector can be provided. This allows to further reduce signal contributions from light passing through melanin rich skin layers and may help to reduce disparate bias.

The photoplethysmography device can be one of (a) a nasal (SpO2) sensor, in particular nasal alar (SpO2) sensor, (b) a finger clip sensor, or (c) an ear clip sensor. A classical sensor for pulse oximetry a is finger clip SpO2 sensor, also briefly referred to as finger clip sensor. An ear clip sensor or a nasal alar sensor can advantageously provide a strong consistent signal even in case of poor perfusion or in shock condition. Moreover, these sensors can be less susceptible to motion artifacts.

### BRIEF DESCRIPTION OF THE DRAWINGS

These and other aspects of the invention will be apparent from and elucidated with reference to the embodiment(s) described hereinafter. In the following drawings:
Fig. 1 shows an exemplary embodiment of a system for determining vital sign information of a subject, in particular for blood oxygen saturation measurement;
Fig. 2 shows a schematic diagram of a device for determining vial sign information of a subject;
Fig. 3 shows a further exemplary embodiment of a device for acquiring a biosignal of a subject as a nasal alar sensor.

### DETAILED DESCRIPTION OF THE EMBODIMENTS

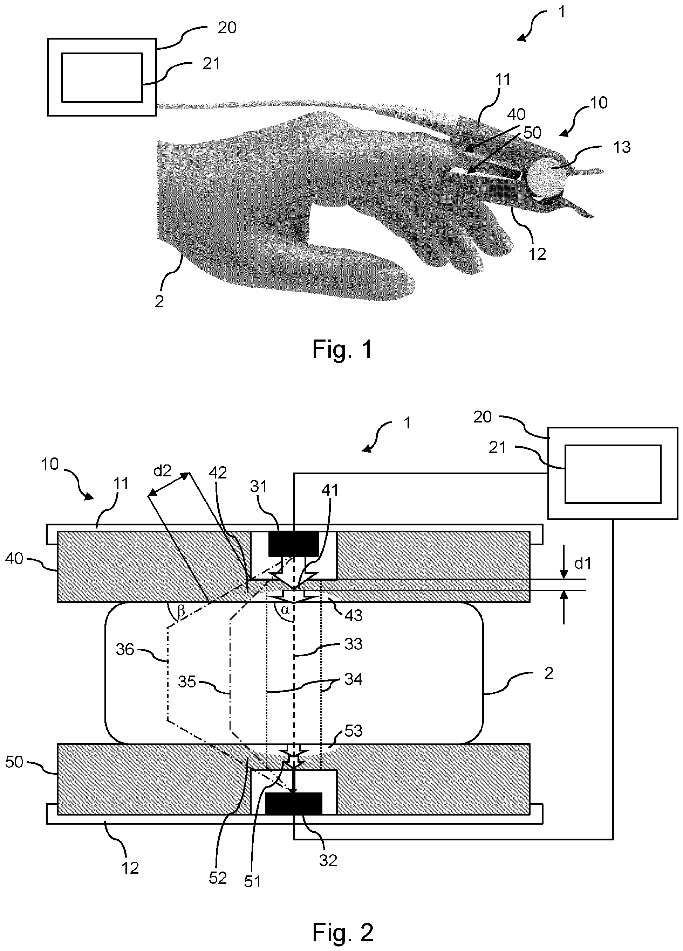

Fig. 1 shows an exemplary embodiment of a system 1 for determining vital sign information of a subject 2, in particular for heart rate monitoring and blood oxygen saturation measurement. The system is therein denoted in its entirety with reference numeral 1.

The system 1 comprises a photoplethysmography device 10 for acquiring a biosignal of the subject and an analyzer 20. The photoplethysmography device 10 is wired or wirelessly coupled to the analyzer 20. The analyzer is configured to determine vital sign information of the subject, such as a pulse rate, blood oxygen saturation and/or respiratory information, from one or more detection signals provided by the photoplethysmography device 10. The analyzer 20 can optionally comprise a display 21 for showing the vital sign information. The analyzer 20 can be implemented as a patient monitor or part thereof.

In the embodiment shown in Fig. 1, the photoplethysmography device 10 is implemented in form of a finger clip sensor. The photoplethysmography device 10 is attached to a finger of the subject 2 for analysis of finger tissue. However, other implementations such as a nasal alar sensor or an ear clip sensor are also possible. In the embodiment shown in Fig. 1, the photoplethysmography device 10 is a transmission type photoplethysmography device. Accordingly, the photoplethysmography device 10 comprises a first portion 11 and second portion 12 that are adapted to be arranged on opposite sides of the tissue of the subject 2 to be analyzed. The tissue can for example be finger tissue, nasal alar tissue or ear lobe tissue. However, the present disclosure is not limited thereto.

Referring again to Fig. 1, the first portion 11 and the second portion 12 are connected by a hinge or connecting piece 13 that is adapted to hold them in abutment against the body part of the subject 2 with the tissue to be measured. For contacting the body part of the subject 2, photoplethysmography device 10 comprises a tissue interface 40, 50.

Fig. 2 shows a more detailed schematic diagram of a system 1 with an analyzer 20 and a device 10 for determining vial sign information of a subject 2. The device 10 comprises a light source 31 configured to emit light signals at one or more wavelengths into tissue of the subject 2. The device further comprises a light detector 32 configured to detect light after travelling from the light source 31 through the tissue of the subject 2 to the light detector 32 along a light path 33 and generate one or more detection signals based on the detected light. In a transmission type PPG device, the light source 31 and light detector 32 are arranged on opposite sides of a tissue region of the subject 2, for example on opposite sides of a finger, ear lobe or nasal alar. A direct or line-of-sight light path 33 from the light source 31 through the tissue of the subject 2 to the light detector 32 can be provided. Both the light source 31 and the light detector 32 can be coupled to an analyzer 20 configured to determine vital sign information of the subject from the one or more detection signals.

The device 10 comprises a tissue interface 40 comprising a first tissue interface region 41 covering the light source 31 along the light path 33 and adapted to transmit light at the one or more wavelengths. In the example shown in Fig. 2, the first tissue interface region 41 can thus be a tissue interface region covering the light source 31 on the direct light path 33 from the light source 31 to the light detector 32, and with limited spatial extent around the direct light path 33 from the light source 31 to the light detector 32, for example within the boundary lines 34. The first tissue region 41 region can refer to the tissue interface region located between the light source 31 and the side of tissue interface 40 adapted to contact the skin of the subject, in particular along the direct or line of sight light path 33 from the light source 31 to the light detector 32.

The tissue interface 40 further comprises a second tissue interface region 42 at least partially surrounding the first tissue interface region 41 and adapted to provide relatively less transmission or attenuate light at the one or more wavelengths. However, instead of providing a tissue interface with two components, wherein one is a window, e.g. a glass window, and the other component is a surrounding cushion from a different material, the tissue interface 40 comprising the first tissue interface region 41 and the second tissue interface region 42 is a monolithic component made from one single material. The optical characteristics of the tissue interface 40 are specifically adapted such that, even though the tissue interface 40 is a monolithic component made from one single material, the first tissue interface region 41 is sufficiently transmissive to transmit enough light for the PPG measurement at the one or more wavelengths along the direct light path 33 from the light source 31 to the light detector 32. The first tissue interface region 41 covers the light source 31, in particular a central portion of the light source 31 along the light path, e.g. between boundaries 34. The first tissue interface region can have a width around the direct light path 33 and in a direction orthogonal to the direct light path 33 of for example ±4 mm, in particular of ±3 mm, in particular of ±2 mm, in particular of ± 1 mm. The boundary 34 of the first tissue interface region can be defined as the region, wherein the transmittance is reduced by at least 30%, in particular by at least 50%, in particular by at least with respect to a maximum transmittance through the first tissue interface region, in particular with respect to a transmittance along the direct light path 33 between the light source 31 and the light detector 32. The second tissue interface region 42 at least partially surrounding the first tissue interface region 41 is attenuating light at the one or more wavelengths outside said (direct) light path 33 to a certain extent. In other words, the second tissue interface region is adapted to have relatively less transmission through e.g. light path 35, 36 or a light path outside or along boundary 34 compared to light path 33. Accordingly, contributions from angled or stray light 35, 36 which may travel though the upper skin layers with high skin pigmentation can thus be reduced, thereby favorably reducing disparate bias in blood oxygen saturation measurements. In particular, a material of the tissue interface 40 can be a very high absorbent, semitransparent, non-scattering, optically neutral and/or controlled density material.

In addition or in the alternative, the receiver side with the light detector 32 can be adapted accordingly. The device 10 comprises a tissue interface 50 comprising a first tissue interface region 51 covering the light detector 32 along the light path 33 and adapted to transmit light at the one or more wavelengths. In the example shown in Fig. 2, the first tissue interface region 51 can thus be a tissue interface region directly covering of the light detector 32 on the direct light path 33 from the light source 31 to the light detector 32, and with limited spatial extent around the direct light path 33 from the light source 31 to the light detector 32, for example within the boundary lines 34. The second tissue region 51 region can refer to the tissue interface region located between the light detector 32 and the side of tissue interface 40 adapted to contact the skin of the subject, in particular along the direct or line of sight light path 33 from the light source 31 to the light detector 32. The tissue interface 50 further comprises a second tissue interface region 52 at least partially surrounding the first tissue interface region 51 and adapted to provide relatively less transmission or attenuate light at the one or more wavelengths. However, instead of providing a tissue interface with two components, wherein one is a window, e.g. a glass window, and the other component is a surrounding cushion from a different material, the tissue interface 50 comprising the first tissue interface region 51 and the second tissue interface region 52 is a monolithic component made from one single material.

The material of the tissue interface 40, 50 is, in the shown embodiment, a material having a transmittance of less than 70 %, in particular of less than 50 %, in particular of less than 30 % at 2 mm material thickness at the one or more wavelengths of the light source 31. In addition, the material of the tissue interface 40, 50 can have a transmittance of at least 5 %, in particular of at last 10%, at 2 mm material thickness at the one or more wavelengths of the light source 31. In addition or in the alternative, the material of the tissue interface 40, 50 is a material having a haze of less than 50 %, in particular of less than 20 %, in particular of less than 5 %, in particular of less than 2 % at 2 mm material thickness.

The light source 31 can be configured to emit light signals at a first red wavelength, for example at a wavelength of 662 nm, and a second infrared wavelength, for example at 880 nm, into the tissue of the subject 2, wherein the material of the tissue interface 40, 50 is a material, wherein the transmittance at the first red wavelength and at the second infrared wavelength deviates by no more than ±50 %, in particular by no more than ±20 %, in particular by no more than ±5 % from one another.

For example, the material of the respective first tissue interface region 41, 51 and the second tissue interface region 42, 52 can be a specifically adapted tinted material, in particular a material having grey color, with specifically adapted optical properties as described herein.

Referring again to Fig. 2, the tissue interface 40, 50 is adapted such that an optical path length through the first tissue interface region 41, 51 is shorter than through the second tissue interface region 42, 52. As shown in Fig. 2, a material thickness d1 along the direct line of sight light path 33 between the light source 31 and the light detector 32 is shorter than a material thickness d2 along the peripheral light paths 35 or 36. This aspect of having different path lengths can be further enhanced by providing a recess or pocket 43, 53 wherein the light source 31 and/or the light detector 32 is arranged at the pocket 43 ,53 in the tissue interface 40,50, wherein said pocket 43, 53 is adapted to provide a minimum, i.e. reduced, material thickness d1 at a center of the light source 31 and/or at a center of the light detector 32. Basically the one or more pockets 43, 53 are adapted and arranged to further reduce absorption along the direct light of sight path 33. In other words, the recesses or pockets 43, 53 can be arranged at a tissue side of the respective tissue interface 40, 50. The recesses or pockets 43, 53 can be arranged along the direct light of sight path 33 between the light source 31 and the light detector 32. The recesses or pockets 43, 53 can be arranged with a minimum thickness of the respective tissue interface at said pocket 43, 53 at a center of the light source 31 and/or at a center of the light detector 32. It will be appreciated that at a center of the light source or of the light detector can refer to a point within a ±4 mm radius, i.e. some alignment tolerances can be acceptable.

Further, the respective light source or light detector side tissue interface 40, 50 can be adapted to provide an angle-dependent transmission (an angle-dependent absorption) for light emitted by the light source 31 and/or received by the light detector 32. As illustrated in Fig. 2, the material thickness to be passed by light on the direct light path 33 at angle α is much lower than on the peripheral light path 36 at angle β, here at 60° with respect to the direct light path. Accordingly, in view of a rather strongly absorbing material of the tissue interface 40, 50 the light intensity in the periphery is reduced rather substantially. In other words, the transmission may be maximum at the direct light path. The transmission may be specifically adapted to decrease with incidence angle. Hence, light that would penetrate the upper skin layers with high melanin content at shallow angles and thereby having the risk of strongly contributing to disparate bias, can be reduced. In particular, as shown in the example in Fig. 2, the device 10 can be adapted such that the absorption increases progressively with emission or incidence angle. This can allow some adjustment tolerance when placing the device 10 at the subject 2. For example, with reference to the embodiment of Fig. 1, different opening angles of the hinge or connecting piece 13 can be tolerated to a certain extent.

Referring again to the angles shown in Fig. 2, the respective tissue interface 40, 50 can be configured such that light emitted from the light source 31 and/or received by the light detector 32 at an angle of 45°, in particular at an angle of 60°, with respect to the direct optical path 33 from the light source 31 through the tissue of the subject 2 to the light detector 32 can be attenuated by the tissue interface 40 ,50 by at least 30%, in particular by at least 50%, in particular by at least 70%.

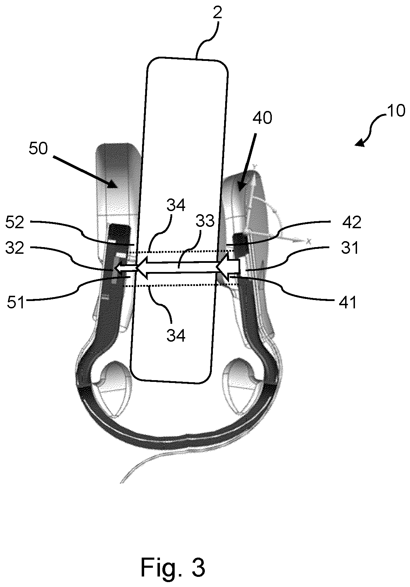

Fig. 3 shows a further exemplary embodiment of a photoplethysmography device 10 for acquiring a biosignal of a subject 2 in the form of a nasal alar sensor. The device 10 again comprises a light source 31 configured to emit light signals at one or more wavelengths into tissue of the subject 2 and a light detector 32 configured to detect light after travelling from the light source 31 through the tissue of the subject 2 to the light detector 32 along a light path 33 and generate one or more detection signals based on the detected light.

The photoplethysmography device 10 comprises a light source side tissue interface 40 and a light detector side tissue interface 50. In other words, a tissue interface 40 is arranged at the side of the light source 31 and a tissue interface 50 is arranged at the side of the light detector 32.

The tissue interface 40 comprises a first tissue interface region 41 covering the light source 31 along the light path 33 and is adapted to transmit light at the one or more wavelengths. The tissue interface 40 further comprises a second tissue interface region 42 at least partially surrounding the first tissue interface region 41 and is adapted to provide relatively less transmission or attenuate light at the one or more wavelengths. The tissue interface 40 comprising the first tissue interface region 41 and the second tissue interface region 42 is a monolithic component made from the same one single material. The dashed lines 34 again illustrate different regions of the tissue interface 40.

In addition or in the alternative, the tissue interface 50 comprises a first tissue interface region 51 covering the light detector 32 along the light path 33 and is adapted to transmit light at the one or more wavelengths. The tissue interface 50 further comprises a second tissue interface region 52 at least partially surrounding the first tissue interface region 51 and is adapted to provide relatively less transmission or attenuate light at the one or more wavelengths. The tissue interface 50 comprising the first tissue interface region 51 and the second tissue interface region 52 is a monolithic component made from the same one single material. The dashed lines 34 again denote exemplary different regions of the tissue interface 50.

As illustrated by the three arrows of reducing thickness in Fig. 3, and similarly in Fig. 2, even along the direct optical path 33 from the light source 31 to the detector 32, the light source side tissue interface 40 and the light detector side tissue interface 50 can be made of a material that has a rather high absorption. It may seem counterintuitive to also cover the light source 31 and/or light detector 32 with a tissue interface material with high absorption. However, this allows to provide a tissue interface that is sufficiently transmissive to transmit enough light for the PPG measurement in a first (or central) tissue interface region 41, 51, while at the same time being sufficiently attenuating stray light or angled light in a second tissue interface region 42, 52 at least partially surrounding the first tissue interface region. Thereby, disparate bias in blood oxygen saturation measurements can be reduced while at the same time improving user comfort and reducing manufacturing costs. Note that the first tissue interface region 41 can refer to a central tissue interface region covering the light source 31. In addition or in the alternative, the first tissue interface region 51 can refer to a central tissue interface region covering the light detector 32.

It should be noted that a tissue interface 40, 50 as described herein may be provided at the respective light source 31, at the respective light detector 32, or both at the light source 31 and at the light detector 32. Moreover, a combined tissue interface for both the light source 31 and the light detector 32 can be provided. For example, for the case of a finger clip PPG device, a combined tissue interface can be provided into which the finger of the subject can be inserted, such as a tubular tissue interface. Similarly, also for the case of a nasal alar nor ear lobe, a combined tissue interface can be proved that folds around the nasal alar or ear lobe such that the light source 31 and light detector 32 can be positioned on opposite sides for a transmission type PPG measurement.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Photoplethysmography device (10) for acquiring a biosignal of a subject (2), in particular for blood oxygen saturation measurement, the device comprising:
- a light source (31) configured to emit light signals at one or more wavelengths into tissue of the subject (2);
- a light detector (32) configured to detect light after travelling from the light source (31) through the tissue of the subject (2) to the light detector (32) along a light path (33) and generate one or more detection signals based on the detected light;
- wherein the device comprises a tissue interface (40, 50) comprising a first tissue interface region (41, 51) covering the light source (31) and/or the light detector (32) along the light path (33) and adapted to transmit light at the one or more wavelengths, and a second tissue interface region (42, 52) at least partially surrounding the first tissue interface region (41, 51);
wherein the second tissue interface region (42, 52) is adapted to provide less light transmission than the first tissue interface region (41, 51) at the one or more wavelengths, and;
wherein the tissue interface (40, 50) comprising the first tissue interface region (41, 51) and the second tissue interface region (42, 52) is a monolithic component made from one single material.

2. Photoplethysmography device according claim 1, wherein the tissue interface (40, 50) is adapted such that an optical path length through the first tissue interface region (41, 51) is shorter than through the second tissue interface region (42, 52).

3. Photoplethysmography device according to any of the preceding claims, wherein a thickness of the second tissue interface region (42, 52) is larger than a thickness of the first tissue interface region (41, 51).

4. Photoplethysmography device according to any of the preceding claims, wherein the tissue interface (40, 50) is a monolithic tissue interface cushion and wherein the light source (31) and/or light detector (32) is embedded in said cushion.

5. Photoplethysmography device according to any of the preceding claims, wherein the material of the tissue interface (40, 50) is a material having a transmittance of less than 70 % at 2 mm material thickness at the one or more wavelengths of the light source (31); in particular having a transmittance of less than 50 % at 2 mm material thickness at the one or more wavelengths of the light source (31).

6. Photoplethysmography device according to claim 5, wherein the material of the tissue interface (40, 50) is a material having a transmittance of at least 5 % at 2 mm material thickness at the one or more wavelengths of the light source (31); in particular having a transmittance of at least 10% at 2 mm material thickness at the one or more wavelengths of the light source (31).

7. Photoplethysmography device according to any of the preceding claims, wherein the light source (31) is configured to emit light signals at a first red wavelength and a second infrared wavelength into the tissue of the subject (2), wherein the material of the tissue interface (40, 50) is a material, wherein the transmittance at the first red wavelength and at the second infrared wavelength deviates by no more than ±50%, in particular by no more than ±20%, in particular by no more than ±5% from one another.

8. Photoplethysmography device according to any of the preceding claims, wherein the material of the tissue interface (40, 50) is a material having a haze of less than 50%, in particular of less than 20%, in particular of less than 5%, in particular of less than 2% at 2 mm material thickness.

9. Photoplethysmography device according to any of the preceding claims, wherein the tissue interface (40, 50) is adapted to provide an angle-dependent transmission for light emitted by the light source (31) and/or received by the light detector (32), wherein the transmission decreases progressively with emission or incidence angle.

10. Photoplethysmography device according to any of the preceding claims, wherein the light source (31) and/or the light detector (32) is arranged at a pocket (43, 53) in the tissue interface (40, 50), wherein said pocket comprises a sloped sidewall and is adapted to provide a minimum material thickness at a center of the light source (31) and/or at a center of the light detector (32).

11. Photoplethysmography device according to any of the preceding claims, wherein the material of the first tissue interface region (41, 51) and the second tissue interface region (42, 52) is the same tinted material, in particular a tinted material having black to grey color, in particular the same tinted material.

12. Photoplethysmography device according to any of the preceding claims, wherein the device is a transmission type photoplethysmography device.

13. Photoplethysmography device according to any of the preceding claims, wherein the device is one of (a) a nasal sensor, in particular nasal alar sensor, (b) a finger clip sensor, or (c) an ear clip sensor.

14. Tissue interface (40, 50) for a photoplethysmography device (10) according to any of the preceding claims, the tissue interface (40, 50) comprising:
a first tissue interface region (41, 51) adapted to cover the light source (31) and/or the light detector (32) along the light path (33) and adapted to transmit light at the one or more wavelengths, and;
a second tissue interface region (42, 52) at least partially surrounding the first tissue interface region (41, 51);
wherein the second tissue interface region (42, 52) is adapted to provide less light transmission than the first tissue interface region (41, 51) at the one or more wavelengths, and;
wherein the tissue interface (40, 50) comprising the first tissue interface region (41, 51) and the second tissue interface region (42, 52) is a monolithic component made from the one single material having a defined optical non-scattering absorption characteristic for the one or more wavelengths.

15. System (1) for determining vital sign information of a subject (2), in particular for blood oxygen saturation measurement, the system comprising:
- a photoplethysmography device (10) according to any of claims 1 to 13; and
- an analyzer (20) configured to determine vital sign information of the subject (2) from the one or more detection signals.
